Europäisches Patentamt

European Patent Office

Office européen des brevets

(11) Publication number: **0 121 578**
**B1**

## (12) EUROPEAN PATENT SPECIFICATION

(45) Date of publication of patent specification: **27.07.88**

(21) Application number: **83103280.0**

(22) Date of filing: **02.04.83**

(51) Int. Cl.⁴: **A 61 M 5/16,** G 01 F 13/00,
G 01 F 3/00

(54) Liquid-flow measuring device and method particularly useful for drip chambers.

(43) Date of publication of application:
**17.10.84 Bulletin 84/42**

(45) Publication of the grant of the patent:
**27.07.88 Bulletin 88/30**

(84) Designated Contracting States:
**AT BE CH DE FR GB IT LI NL SE**

(56) References cited:
DE-A-2 221 564
FR-A- 227 970
FR-A-2 341 841
GB-A-2 097 679
GB-A-2 102 566
US-A-3 030 954
US-A-4 105 028

(73) Proprietor: **Leibinsohn, Saul**
**11 Olei Hagardom Street**
**Rishon Lezion (IL)**

(72) Inventor: **Leibinsohn, Saul**
**11 Olei Hagardom Street**
**Rishon Lezion (IL)**

(74) Representative: **Modiano, Guido et al**
**MODIANO, JOSIF, PISANTY & STAUB Modiano**
**& Associati Via Meravigli, 16**
**I-20123 Milan (IT)**

Courier Press, Leamington Spa, England.

## Description

The present invention relates to liquid-flow measuring devices. The invention is particularly useful in drip chambers for infusion administration sets, and is therefore described below with respect to this application.

Drip chambers for infusion administration sets commonly include a housing having an internal chamber for accumulating the liquid dripping thereinto by gravity introduced via an inlet port at one end, and for feeding the liquid therefrom via an outlet port at the opposite end. Many applications of such drip chambers require the liquid to be administered at a predetermined flow rate and/ or for a predetermined quantity. The known arrangements, however, are not very precise with respect to the flow rate or the total quantity of the liquid being administered; moreover they require frequent monitoring by a nurse, thereby adding to her already heavy work-load.

One known arrangement comprising the features of the first part of claim 1 is described in GB—A—2,102,566.

According to the present invention, there is provided a liquid-flow measuring device, particularly useful as a drip chamber for infusion administration sets, including a housing (30; 130; 230; 430) having an internal chamber (32; 132; 232; 432a) for accumulating the liquid, said housing having an inlet port (11; 111; 211) situated at one end of said housing for introducing the liquid thereto, said inlet port comprising a first bore (12; 112) leading to a second bore (22; 122) at a juncture between the two bores, the diameters of the two bores being such that drips of liquid form at the juncture of said bores, each drip forming a liquid column in said second bore (22; 122), which liquid column moves downwardly by gravity and breaks away into said chamber, the length of each column and the number of columns per unit of time thereby providing a measurement of the rate of flow of the liquid through said device, said housing further having an outlet port (42; 142; 233; 433) at the opposite end thereof for feeding the liquid out of the chamber, characterized in that said housing further includes an air passage (26, 28; 126, 128) leading from said chamber (32; 132) to said juncture of the first and second bores.

The characterising feature, which is not present in GB—A—2,102,556, permits the liquid column in the second bore to grow downwardly by gravity and to break away into the chamber before forming a drop at the bottom of the second bore. Moreover, providing the air passage from the chamber, rather than from the outside, prevents the entry of foreign contaminants into the liquid.

According to another aspect of the invention there is provided a method of measuring liquid-flow particularly in a drip chamber for infusion administration sets of the type including a housing (30, 130, 230, 430) having an internal chamber (32, 132, 232, 432a) for accumulating liquid, wherein said housing has an inlet port (11, 111, 211) situated at one of its ends for introducing liquid thereto, and wherein the inlet port comprises a first bore (12, 112) leading to a second bore (22, 122) at a juncture between the two bores, said method comprising the steps of:

feeding liquid from said bore into said second bore;

arranging the diameters of the two bores such that drips of liquid form at the juncture of said bores, each drip forms a liquid column in said second bore, and the liquid column moves downwardly by gravity and breaks away into said chamber;

measuring the length of each column and the number of columns per unit of time to provide a measurement of the rate of flow through the drip chamber; and

providing in said housing an outlet port (42, 142, 233, 433) at the opposite end thereof for feeding liquid out of the chamber, said method being characterized by providing in said housing an air passage (26, 28, 126, 128) leading from said chamber (32, 132) to said juncture of the first and second bores.

The invention is herein described, by way of example only, with reference to the accompanying drawings wherein:

Fig. 1 is a longitudinal sectional view illustrating one form of liquid-flow measuring device constructed in accordance with the invention, the device being embodied in a drip chamber for infusion administration sets;

Fig. 2 is a longitudinal section of the upper end of the device of Fig. 1 but illustrating a modified construction;

Fig. 3 is an enlarged view of a composite sleeve included in the modification of Fig. 2;

Fig. 4 is a fragmentary sectional view along lines IV—IV of Fig. 3;

Fig. 5 is a longitudinal sectional view of the lower end of the device of Fig. 1 but illustrating a further modification;

Fig. 6 is a block diagram of an electrical measuring circuit that may be used with the illustrated device;

Fig. 7 is a longitudinal sectional view illustrating another form of liquid-flow measuring device constructed in accordance with the invention;

Fig. 8 is a fragmentary view illustrating a modification in the device of Fig. 7; and

Fig. 9 illustrates a further device constructed in accordance with the invention.

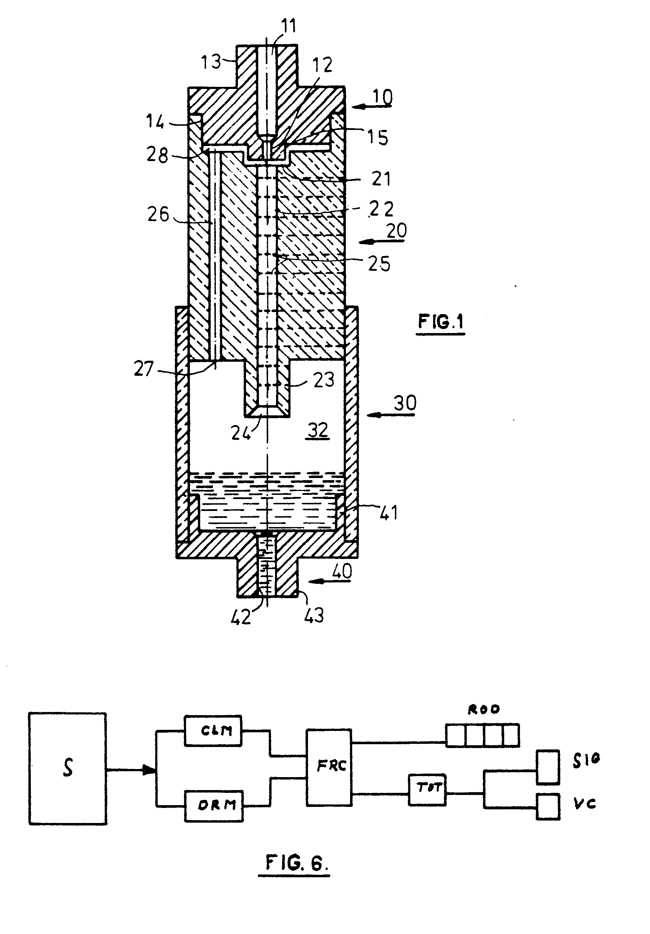

The liquid-flow measuring device illustrated in Fig. 1 is particularly useful in drip chambers for infusion administration sets. Generally speaking, the device comprises a housing including four main sections attached together, namely a first section 10 including the inlet to the device, a second section 20 including means enabling one to observe the liquid flow such as to provide an indication of the flow rate, a third section 30 defining an internal chamber for accumulating the liquid dripping thereinto by gravity, and a fourth section 40 including an outlet port for feeding the liquid from the latter chamber out of the device. The foregoing parts are of circular cross-section so that the housing is of generally cylindrical configuration. The parts are assembled together by friction fits to facilitate the quick assembly and disassembly of the device.

More particularly, housing section 10 includes the inlet port defined by a longitudinal bore 11 of reduced diameter at its inner end as shown at 12. The outer end of housing section 10 is formed with an external stem 13, preferably tapered, to permit its quick attachment to a flexible tube (not shown), usually of elastomeric (e.g., natural or synthetic rubber) material, leading to the container of the liquid to be administered by the infusion device. The inner end 14 of housing section 10 is similarly tapered for frictional attachment to housing section 20, and includes a central stem 15 projection into a corresponding central recess 21 in housing section 20. Bore 12 passes centrally through stem 15.

Housing section 20 is also formed with a central bore 22 extending axially therethrough in alignment with, but of larger diameter than, bore 12. The opposite end of bore 22 passes through a stem 23 projecting below the lower face of housing section 20. The end of bore 22 is enlarged as shown at 24.

Housing section 20 is further provided with a plurality of graduation markings 25 along bore 22, for purposes described below. This housing section is further formed with another bore 26 extending axially through the housing section but spaced laterally from central bore 22. Bore 26 does not pass through stem 23, so that the lower end 24 of bore 22 is disposed at a lower elevation than the lower end 27 of bore 26.

Bore 26 serves as an air return passage to the junctures between bores 12 and 22. Thus, the upper end of bore 26 communicates with the juncture between bores 12 and 22 via air passages, which may include an air space 28 between the two housing sections 10 and 20, and/or recesses formed on one or both contacting faces of these housing sections.

Housing section 30 defines an internal chamber 32 with housing sections 20 and 40. Thus, housing section 30 is in the form of a cylindrical sleeve frictionally fittable at one end to the outer surface of housing section 20, and at the opposite end to an axial flange 41 formed in the inner face of housing section 40.

In addition to the attaching flange 41, housing section 40 is formed with a central bore 42 serving as the outlet port for the liquid accumulating within chamber 32, and with a tapered stem 43 for attachment to the infusion line (not shown) leading to the infusion needle.

The relative diameters of bore 12 at the terminal end of the inlet port 11, and bore 22 within housing section 20, are such that the liquid flowing through the inlet port 11 and bore 12 forms a liquid column in bore 22, which liquid column moves until it drops by gravity into chamber 32. Bore 26, and the air passage 28 leading to the juncture of bore 12 with bore 22, provide an air return permitting the liquid column to move within bore 22 and to drop by gravity into chamber 32. Housing section 20 is of transparent material to permit visual observation, via the graduation markings 25, of the length of the liquid column growing in bore 22. Thus, bore 12 serves as a liquid metering element, and bore 22 serves as a liquid measuring element, enabling the user, by observing the length of the liquid column formed in bore 22 before dropping into chamber 32, and also the rate at which such columns drop into chamber 32, to determine the rate of flow of the liquid through the device.

As one example, for infusion solutions of 0.9% NaCl and 5% or 10% glucose, with the solution source level about 1.20 meters above the patient, the diameter of bore 12 may be about 1.5 mm, and that of bore 22 may be from 2—4 mm (e.g., 3 mm), depending on the flow rate desired.

Since the outlet end 24 of bore 22 is at a lower elevation within the liquid chamber 32 than the inlet end 27 of the air-return bore 26, this decreases the possibility that liquid flowing through bore 22 may clog the inlet end 27 of the air return bore 26. In addition, since the outlet end of bore 12 is formed through stem 15, this decreases the possibility that liquid flowing through bore 12 to form the column moving within bore 22 may clog the air-return passages 28 leading to the juncture between bores 12 and 22, which air return is necessary to permit the liquid column to move within bore 22 and to drop into chamber 32. In order to further decrease the possibility of clogging the air return path, the outer surface of stem 15 and/or the inner face of the recess 21 receiving same, may be coated with a hydrophobic substance, such as silicone or wax.

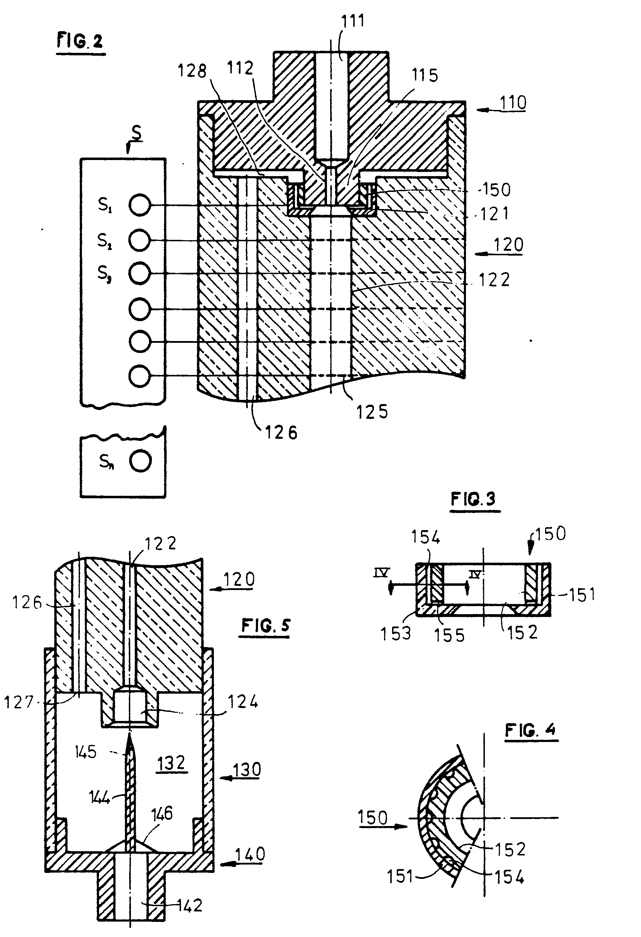

Fig. 2 illustrates a modification that may be embodied in the device, particularly at the juncture between the upper housing section therein designated 110, and the adjacent housing section therein designated 120. Thus, in the modification illustrated in Fig. 2, a composite cylindrical sleeve 150 is interposed between stems 115 in housing section 110, and the corresponding recess 121 in housing section 120. Sleeve 150 is of a special construction to provide a non-cloggable air-return passage from bore 126 in housing section 120 to the juncture between bore 112 at the terminal end of the inlet port 111 in housing section 110, and the upper end of bore 122 in housing section 120.

The structure of sleeve 150 is more particularly illustrated in Figs. 3 and 4, wherein it will be seen that it includes an outer sleeve section 151 coaxially receiving an inner sleeve section 152. The lower end of the outer sleeve section 151 is formed with a apertured end wall 153 engaged by the lower face of the inner sleeve section 152. In addition, the outer face of sleeve section 152 is formed with a plurality of axially-extending, circumferentially-spaced recesses 154 leading to radially-extending recesses 155 formed in the lower face of sleeve section 152.

As shown particularly in Fig. 2, the lower end of stem 115 in housing section 110 seats against the inner face of the apertured end wall 153 of sleeve section 151 such as to provide a space 128 between the confronting lower face of housing section 110 and upper face of housing section 120. This space 128 is in the air-return path from bore 126, which path also includes recesses 154 and 155 in the composite sleeve 150 leading to the juncture between bore 112 in housing section 110, and bore 122 in housing section 120. This air-return path thus permits the liquid flowing through bore 112 to form a moving column within bore 122, as described above with respect to Fig. 1, without danger of clogging.

Fig. 5 illustrates a modification that may be provided at the lower end of the device illustrated in Fig. 2 to further assure that the liquid exiting from the column-measuring bore 122 into chamber 132, formed by housing section 130, will not clog the air inlet opening 127 of the air-return duct path 126. For this purpose, the lower housing section 142 is provided with a vertical stem 144 having a pointed upper end 145 in alignment with the outlet end 124 of bore 122, in order to break or pierce any drop forming at the lower end, and thereby to prevent same from covering or clogging the inlet end 127 of the air-return path 126. Stem 144 is preferably integrally formed with housing section 144 by means of a plurality of spaced radially-extending ribs 146 to provide a passage for the fluid flow from chamber 132 through the outlet port 142.

As described above with respect to Fig. 1, housing section 120 may be formed with a plurality of graduation markings 125 along bore 122 to permit visual observation of the length of the liquid column moving within the bore before dropping into the drip chamber. Thus, by visually observing the length of the vertical column in bore 122 before it drops into the chamber, and the rate at which such vertical columns drop into the chamber, one may easily determined the rate of flow of the liquid through the device. In practice, it would normally be necessary to make the above observations only with respect to the first few vertical columns, since the column length and drop rate will generally remain substantially constant for the complete procedure. The user may therefore be provided with a chart or table converting column length and drop rate to rate of flow per unit time.

For a more precise measurement of the flow rate, the device may be provided with an electrical measuring circuit comprising a sensor circuit S including a plurality of sensors $S_1$, $S_2$ ... $S_n$, each sensor optically aligned with one of the graduation markings 125 along the length of bore 122 as shown in Fig. 2. Thus, the first sensor $S_1$ would be aligned with the juncture line between the lower end of bore 122 and the upper end of bore 122. This sensor can be used to indicate when the liquid column growing within bore 122 drops into the drip chamber since, at this instant, it will sense an interruption in the liquid at this juncture line. The rate of such interruptions would thus indicate the rate at which the vertical columns fall into the drip chamber. In addition, the sensor circuit S may be designed so that as soon as this interruption by sensor $S_1$ is sensed, the length of the column can be determined, by sensing where its bottom edge lies with respect to the remaining sensors. Optical sensors and circuits which may be used for this purpose are known in the art, for example, in optical character or pattern recognition systems, and therefore further details of the construction or operation of such sensors are not deemed necessary herein.

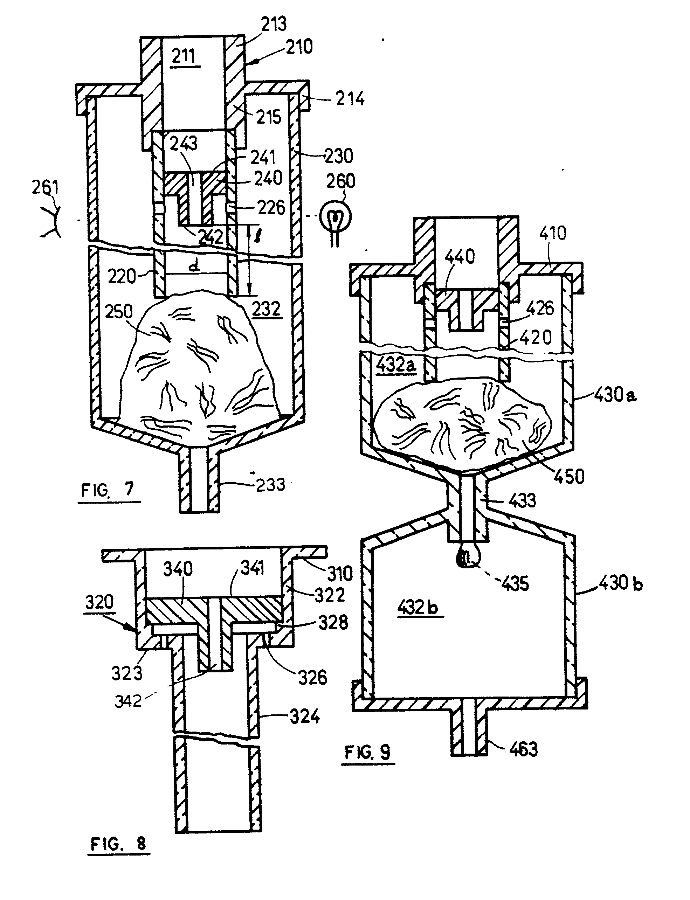

The complete electrical measuring circuit is schematically shown in block diagram form in Fig. 6, wherein it will be seen that the sensors of circuit S are connected to a column-length-measuring circuit CLM and the a drop-rate-measuring circuit DRM. The latter two circuits are connected to a flow-rate calculator FRC which calculates the flow rate from the information received from circuits CLM and DRM. The flow-rate calculator FRC outputs its readings to a readout display ROD to provide a continuous display of the flow-rate, namely the flow quantity, e.g., ml/min. The flow-rate calculator FRC may be also connected to a totalizer TOT which may be preset for a predetermined quantity, so as to control a signalling device SIG providing a signal (visual or audio) when the predetermined quantity has been reached, and/or a valve control circuit VC to terminate the infusion procedure when the predetermined quantity has been reached.

Figs. 7—10 illustrate further devices constructed in accordance with the invention wherein the device includes a column-breaking element located so as to be contacted by the moving liquid column, and to break same, slightly before the liquid column would otherwise break by gravity alone. Such an arrangement thereby prefixes the length of the liquid column before breaking and passing into the drip chamber, so that it is only necessary to count the rate such breakages occur in order to determined the rate of liquid flow.

The device illustrated in Fig. 7 comprises three main sections, designated 210, 220 and 230, respectively. Section 210 is formed with a bore 211, an external stem 213, an annular flange 214, and an internal stem 215. The external stem 213 is adapted to receive the connecting flexible tube through which the administered liquid is fed. Annular flange 214 receives, e.g., by a press-fit, section 230 of generally cylindrical configuration to define a cylindrical housing having an internal chamber 232 for the administered liquid, the opposite end of the housing being formed with an external stem 233 to be connected to the tube outletting the administered liquid. Internal stem 215 of section 210 receives, e.g., also by friction fit, section 220 which is in the form of a hollow sleeve through which the administered liquid flows into chamber 232.

Hollow sleeve 220 is formed with one or more (e.g., a circular array) of openings 226 near its upper end. An apertured member 240, of hydrophobic material non-wettable by the administered liquid, is received in the upper end of the hollow sleeve 220 above openings 226. The hydrophobic member 240 is formed with an enlarged head 241 press-fitted against the inner face of hollow sleeve 220, and with a depending stem 242 of smaller diameter than its head 241, the arrangement being such that stem 242 is spaced radially inwardly of the inner face of sleeve 220 through which openings 226 are formed and depends below these openings. A bore 243 is formed through both the enlarged head 241 and the depending stem 242 of the hydrophobic member 240.

Bore 243 through the hydrophobic member 240 is of smaller diameter than bore 211 of the inlet to the chamber and acts as the metering bore for metering the liquid. The hollow sleeve 220 acts as the column-growing bore in which the liquid column moves by gravity, and openings 226 through sleeve 220 acts as the air-return passages at the juncture of the two bores to permit the liquid column to grow within sleeve 220 until the liquid column would normally drop by gravity into chamber 232.

In the arrangement illustrated in Fig. 7, however, sleeve 220 is not of such length as to permit the liquid column to break off by gravity alone and to drop into chamber 232. Instead, the device includes a column-breaking element located so as to be contacted by the liquid column growing within sleeve 220 and to break same slightly before the liquid column would otherwise break by gravity alone. This arrangement thereby prefixes the length of the liquid column before dripping into chamber 232, so that by knowing the dimensions (length and diameter) of hollow sleeve 220 below the bottom of the apertured hydrophobic member 240, one may easily make a determination of the flow rate of the liquid through the device by merely counting the rate at which the liquid column breaks within sleeve 220.

In the arrangement illustrated in Fig. 7, the column-breaking element is in the form of a liquid absorbent material, such as a plastic mesh, e.g., "Dacron" (Reg. TM), a sponge, or a filtering material. This element is disposed within chamber 232 with its upper face engaging the bottom face of the hollow sleeve 220 so as to be contacted by the liquid column moving within the sleeve as soon as it reaches it. When this occurs, the liquid within the sleeve is absorbed by the mesh 250 to break the liquid column growing within sleeve 220, whereupon a new liquid column begins to form.

Thus, by noting, visually or optically, e.g., by the use of an aligned light source 260 and photocell 261 disposed just below the hydrophobic apertured member 240, the rate at which the liquid column breaks, one can determine the rate of flow of the liquid through the device.

As one example, for infusing solutions of 0.9% NaCl and 5% or 10% glucose, with a solution source level about 1.20 meters above the patient, bore 243 may be about 2 mm diameter, hollow sleeve 220 may be 3.15 mm inner diameter (d), and the length (1) if the sleeve 220 from the bottom face of member 240 to the upper face of the mesh 250 may be about 15 mm. This produces about 8 drop/ml. In the above example, the inner diameter (d) may vary from about 2.0 mm to about 3.8 mm to change the size of the vertical columns, i.e., the number if drops/ml.

In the above-described example, sleeve 220 may be clear polyvinyl chloride, and the hydrophobic apertured member 240 may be polypropylene or polytetrafluorethylene. Members 240 may be integrally formed with the hollow sleeve 220, in which case the material should be a clear hydrophobic material, such as polypropylene.

Fig. 8 illustrates a modification in the Fig. 7 construction, wherein the upper housing section, therein designated 310, is integrally formed with the hollow sleeve, therein designated 320. This can be conveniently achieved, e.g., by injection molding. In this construction, the hollow sleeve 320 is enlarged at its upper end 322, and its annular juncture 323 with its lower end 324 of the sleeve is formed with the air-return openings 326. In addition, the apertured hydrophobic member 340 is applied to the upper, enlarged-diameter end 322 of sleeve 320, by press-fitting the member against an inner annular rib 328, so as to space the bottom face of the member's head 341, and also its depending stem 342, from the air-return openings 326.

The construction illustrated in Fig. 8 is used within a cylindrical housing section (not shown) corresponding to housing section 230 in Fig. 7, and otherwise operates in the same manner as described above with respect to Fig. 7.

In the embodiment illustrated in Figs. 7 and 8, the observer actually does not see drops forming within the drip chamber, since the complete formation of the drops is terminated by the contact of the liquid with the absorbent material (250, Fig. 7). In some applications it would be desirable to permit the observer to actually see the drops being formed, so that the operation will more closely resemble the usual operation of drip chambers. In such applications, the arrangement illustrated in Fig. 9 may be used.

The Fig. 9 arrangement includes two drip chambers, one of which is used for measuring the rate of flow of the liquid, and the other of which is used for forming drops which may be observed. Thus, the cylindrical housing in Fig. 9 is constituted of two sections, namely an upper section 430a defining an upper chamber 432a, and a lower section 430b defining a lower chamber 432b. The upper section 430a is constructed in the same manner as described above, e.g., in Fig. 7, for measuring the rate of flow of the liquid, this section includes the hollow sleeve 420 for forming the liquid columns, the hydrophobic metering member 440 at the upper end of the sleeve, the air-return openings 426 through the sleeve, and the column-breaking absorbent mesh, sponge or filter 450 in contact with the lower end of the sleeve. The liquid thus accumulating in the upper chamber 432a flows through its outlet 433 into the lower chamber 432b defined by the lower housing section 430b. As this liquid flows from chamber 432a into chamber 432b, it forms distinct observable drops, as shown at 435, before falling by gravity into chamber 432b from which it is outletted via outlet 463 to the patient. It will be appreciated, however, that whereas the liquid is inletted into the lower chamber 432b in the form of observable drops 435, the tate of formation of these drops is not what indicates the rate of breakage of the liquid, but rather the rate of breakage of the liquid column within the hollow sleeve 420, as described above, is what provides the indication of the liquid flow rate.

**Claims**

1. A liquid-flow measuring device, particularly useful as a drip chamber for infusion administration sets, including a housing having an internal chamber for accumulating the liquid, said housing having an inlet port situated at one end of said housing for introducing the liquid thereto, said inlet port comprising a first bore leading to a second bore at a juncture between the two bores, the diameters of the two bores being such that drips of liquid form at the juncture of said bores, each drip forming a liquid column in said second bore, which liquid column moves downwardly by gravity and breaks away into said chamber, the length of each column and the number of columns per unit of time thereby providing a measurement of the rate of flow of the liquid through said device, said housing further having an outlet port at the opposite end thereof for feeding the liquid out of the chamber, characterized in that said housing further includes an air passage leading from said chamber to said juncture of the first and second bores.

2. The device according to Claim 1, characterized in that at least the portion (20, 30; 120, 130; 220, 230; 320; 430) of the housing containing said second bore (22; 122) is transparent and is provided with graduation markings (25; 125) along the length of said second bore to permit the length of the liquid column moving therein to be visually observed.

3. The device according to Claim 1, characterised in that said device further includes a column-breaking element (144; 250; 450) located at the bottom of said second bore (22; 122) so as to be contacted by the liquid column moving in said second bore, and to break same, when said downwardly moving liquid column reaches the bottom of said second bore, to thereby prefix the length of said liquid column before passing into said chamber (132; 232; 432a).

4. The device according to Claim 3, characterized in that said column-breaking element is a liquid-absorbent element (250; 450) which is contacted by the liquid column to break same.

5. The device according to Claim 3, characterized in that said column-breaking element is a pointed stem (144) disposed in said chamber (132) such as to be contacted by the liquid column when it reaches the lower end of said second bore (122).

6. The device according to Claim 1, characterized in that said second bore is defined by a hollow sleeve (220; 320; 420) whose lower end is disposed within said chamber, the upper end of said hollow sleeve being formed with openings (226; 326; 328; 426) therethrough to define said air-return leading to the juncture of said first and second bores.

7. The device according to Claim 6, characterized in that an axially-apertured hydrophobic member (240; 340; 440) defining said first bore, said hydrophobic member being disposed above said air-return openings (226; 326; 328) to prevent clogging thereof by liquid passing through said first bore.

8. The device according to Claim 7, characterized by a column-breaking element (250; 450) located at the bottom of said second bore so as to be contacted by the liquid column moving in said hollow sleeve to break same when said downwardly-moving liquid column reaches the bottom of said second bore, to thereby prefix the length of said liquid column before passing into said chamber.

9. A method of measuring liquid-flow particularly in a drip chamber for infusion administration sets of the type including a housing having an internal chamber for accumulating liquid, wherein said housing has an inlet port situated at one of its ends for introducing liquid thereto, and wherein the inlet port comprises a first bore leading to a second bore at a juncture between the two bores, said method comprising the steps of:

feeding liquid from said bore into said second bore;

arranging the diameters of the two bores such that drips of liquid form at the juncture of said bores, each drip forms a liquid column in said second bore, and the liquid column moves downwardly by gravity and breaks away into said chamber;

measuring the length of each column and the number of columns per unit of time to provide a measurement of the rate of flow through the drip chamber; and

providing in said housing an outlet port at the opposite end thereof for feeding liquid out of the chamber, said method being characterized by providing in said housing an air passage leading from said chamber to said juncture of the first and second bores.

10. The method according to claim 9, characterised in that the liquid column moving downwardly by gravity in said second bore (22, 122) breaks away into said chamber (32, 132, 232, 432a) before the liquid column reaches the bottom of the second bore.

**Patentansprüche**

1. Durchflußmeßvorrichtung, insbesondere verwendbar für Tropfkammern für die Verabreichung von Infusionen, bestehend aus einem Gehäuse mit einer inneren Kammer zur Aufnahme der Flüssigkeit, welches Gehäuse eine an einem Ende des Gehäuses liegende Einlaßöffnung für die Einführung der Flüssigkeit in dieses besitzt, welche Einlaßöffnung eine erste Bohrung, die zu einer zweiten Bohrung an einer Verbindung zwischen den beiden Bohrungen führt, aufweist, wobei die Durchmesser der beiden Bohrungen derart sind, daß sich Tropfen von Flüssigkeit an der Verbindung der beiden Bohrungen bilden, und jeder Tropfen in der zweiten Bohrung eine Flüssigkeitssäule bildet, welche sich durch die Schwerkraft nach unten bewegt und in die genannte Kammer hinein aufbricht, wobei die Länge einer jeden Säule und die Anzahl der Säulen pro Zeiteinheit ein Maß für die Fließgeshwindigkeit der Flüssigkeit durch die Vorrichtung darstellen und das Gehäuse weiters eine Auslaßöffnung an seinem entgegengesetzten Ende besitzt, um die Flüssigkeit aus der Kammer abzugeben, dadurch gekennzeichnet, daß das Gehäuse weiters einen Luftkanal besitzt, der von der Kammer zur genannten Verbindung der ersten und der zweiten Bohrung führt.

2. Vorrichtung nach Anspruch 1, dadurch gekennzeichnet, daß wenigstens der Teil (20, 30; 120, 130; 220, 230; 320; 430) des Gehäuses, der die Zweite Bohrung (22; 122) enthält, durchsichtig und mit Teilungsmarkierungen (25; 125) entlang der Länge der zweiten Bohrung versehen ist, um eine visuelle Beobachtung der Länge der sich darin bewegenden Flüssigkeitssäule zu ermöglichen.

3. Vorrichtung nach Anspruch 1, dadurch gekennzeichnet, daß sie weiters ein die Flüssigkeitssäule brechendes Element (144; 250; 450) besitzt, das sich am Boden der zweiten Bohrung (22; 122) befindet, um die der sich in der zweiten Bohrung bewegenden Flüssigkeitssäule in Berührung zu gelangen und diese zu brechen, wenn die sich nach unten bewegende Flüssigkeitssäule den Boden der zweiten Bohrung erreicht, um dadurch die Länge der Flüssigkeitssäule vorzufixieren, bevor sie in die genannte Kammer (132; 232; 432a) eintritt.

4. Vorrichtung nach Ansoruch 3, dadurch gekennzeichnet, daß das die Flüssigkeitssäule brechende Element ein Flüssigkeit-absorbierendes Element (250; 450) ist, das mit der Flüssigkeitssäule, um diese zu brechen, in Berührung gebracht wird.

5. Vorrichtung nach Anspruch 3, dadurch gekennzeichnet, daß das die Flüssigkeitssäule brechende Element ein Spitsdorn (144) ist, der in der Kammer (132) derart angeordnet ist, daß er mit der Flüssigkeitssäule in Berührung gelangt, wenn sie das untere Ende der zweiten Bohrung (122) erreicht.

6. Vorrichtung nach Anspruch 1, dadurch gekennzeichnet, daß die zweite Bohrung eine hohle Hülse (220; 320; 420) ist, deren unteres Ende sich in der Kammer befindet, und deren oberes Ende mit durchgehenden Öffnungen (226; 326; 328; 426) versehen ist, um die genannte Luftrückführung zu definieren, die zur Verbindung der ersten und der zweiten Bohrung führt.

7. Vorrichtung nach Anspruch 6, gekennzeichnet durch einen axial offenen hydrophoben Teil (240; 340; 440), der die genannte erste Bohrung definiert und oberhalb der Luftrückführungsöffnungen (226; 326; 328) angeordnet ist, um ein Verstopfen derselben durch die durch die erste Bohrung durchtretende Flüssigkeit zu verhindern

8. Vorrichtung nach Anspruch 7, gekennzeichnet durch ein die Flüssigkeitssäule brechendes Element (250; 450), das am Boden der zweiten Bohrung angeordnet ist, so daß es mit der sich in der hohlen Hülse bewegenden Flüssigkeitssäule in Berührung gelangt, um diese zu brechen, wenn die sich nach unten bewegende Flüssigkeitssäule den Boden der zweiten Bohrung erreicht, um die Länge der Flüssigkeitssäulen vor dem Eintritt in die Kammer vorzufixieren.

9. Verfahren zum Messen des Durchflusses, insbesondere in einer Tropfkammer von Garnituren für die Verabreichung von Infusionen, bestehend aus einem Gehäuse mit einer inneren Kammer zur Aufnahme der Flüssigkeit, welches Gehäuse an einem seiner Enden eine Einlaßöffnung für die Einführung der Flüssigkeit in dieses besitzt, welche Einlaßöffnung eine erste Bohrung, die zu einer zweiten Bohrung an einer Verbindung zwischen den beiden Bohrungen führt, aufweist, welches Verfahren die Stufen umfaßt:

Zufuhr der Flüssigkeit von der ersten Bohrung in die zweite Bohrung;

Wahl der Durchmesser der beiden Bohrungen derart, daß Tropfen der Flüssigkeit an der Verbindund der Bohrungen gebildet werden jeder Tropfen in der zweiten Bohrung eine Flüssigkeitssäule bildet und sich die Flüssigkeitssäule durch Schwerkraft nach unten bewegt und in die Kammer hinein abbricht;

Messen der Länge einer jeden Säule und der Anzahl der Säulen pro Zeiteinheit, um ein Maß für die Fließgeschwindigkeit durch die Tropfkammer zu schaffen; und

Schaffung einer Auslaßöffnung im Gehäuse an dem enthegegensetzten Ende desselben zur Abgabe der Flüssigkeit aus der Kammer, dadurch gekennzeichnet, daß im Gehäuse ein Luftkanal vorgesehen wird, der von der Kammer zur genannten Verbindung der ersten und der zweiten Kammer führt.

10. Verfahren nach Anspruch 9, dadurch gekennzeichnet, daß die sich durch Schwerkraft in der zweiten Bohrung (22, 122) nach unten bewegende Flüssigkeitssäule in die Kammer (32, 132, 232, 432a) hinein abbricht, bevor die Flüssigkeitssäule den Boden der zweiten Bohrung erreicht.

**Revendications**

1. Dispositif pour la mesure du débit d'un liquide, partuculièrement adapté à une chambre d'égouttage pour système de perfusion, comportant un carter présentant une chambre interne pour l'accumulation de liquide, ledit carter présentant un orifice d'entrée suité à une extrémité dudit carter pour y introduire du liquide, ledit orifice d'entrée comprenant un premier alésage conduisant à un second alésage à une jonction entre les deux alésages, les diamètres des deux alésages étant tels que des goutes de liquide se forment à la jonction desdits alésages, chaque goutte formant une colonne de liquide dans ledit second alésage, laquelle colonne de liquide se déplace vers le bas par gravité et s'écoule dans ladite chambre, la hauteur de chaque colonne et le nombre de colonnes par unité de temps fournissant de ce fait une mesure du débit de liquide à travers ledit dispositif, ledit carter présentant de plus un orifice de sortie à l'extrémite opposée de celui-ci pour évacuer le liquide de la chambre, caractérisé en ce que ledit carter comporte, de plus, un passage d'air menant de ladite chambre à ladite jonction des premier et second alésages.

2. Dispositif selon la revendication 1, caractérisé en ce qu'au moins la partie (20, 30; 120, 130; 220, 230; 320; 430) du carter contenant ledit second alésage (22; 122) est transparante et est munie de graduations (25; 125) le long de la hauteur dudit second alésage pour permettre à la hauteur de la colonne de liquide se déplaçant dans celui-ci d'être observée visuellement.

3. Dispositif selon la revendication 1, caractérisé en ce que le dispositif comporte de plus un élément pour rompre le colonne (144; 250; 450) situé à l'extrémité inférieur dudit second alésage (22; 122) afin de venir au contact de la colonne de liquide se déplaçant dans ledit second alésage, et de rompre celle-ci, quand ladite colonne de liquide se déplaçant vers le bas atteint l'extrémité inferieure dudit second alésage, pour ainsi préfixer la hauteur de ladite colonne de liquide avant le passage dans ladite chambre (132; 232; 432a).

4. Dispositif selon la revendication 3, caractérisé en ce que ledit élément pour rompre la colonne est un élément absorbant de liquide (250; 450) qui vient en contact avec la colonne de liquide pour la rompre.

5. Dispositif selon la revendication 3, caractérisé en ce que l'élément pour rompre le colonne est une tige pointue (144) disposée dans ladite chambre (132) de telle sorte qu'elle vient au contact de la colonne de liquide quand elle atteint l'extrémité inférieure dudit second alésage (122).

6. Dispositif selon la revendication 1, caractérisé en ce que ledit second alésage est défini par un manchon creux (220; 320; 420) dont l'extrémité inférieure est disposée à l'intérieur de ladite chambre, l'extrémiré supérieure dudit manchon creux étant pourvue d'ouvertures (226; 326; 328; 426) pour définir le retour d'air menant à la jonction desdits premier et second alésages.

7. Dispositif selon la revendication 6, caractérisé par un élément hydrophobe ouvert axialement (240; 340; 440) définissant ledit premier alésage, ledit élément hydrophobe étant disposée au-dessus des ouvertures de retour d'air (226; 326; 328) pour éviter l'obstruction de celles-ci par le liquide transversant ledit premier alésage.

8. Dispositif selon la revendication 7, caractérisé par un élément pour rompre la colonne (250; 450) situé à l'extrémite inférieure dudit second alésage afin de venir en contact avec la colonne de liquide se déplaçant dans la manchon creux pour la rompre quand la colonne de liquide se déplaçant vers le bas atteint l'extrémité inférieure dudit second alésage, pour de ce fait préfixer la hauteur de ladite colonne de liquide avant son passage dans ladite chambre.

9. Procédé pour le mesure du débit d'un liquide particulièrement adapté à une chambre d'égouttage de sustème de perfusion, du type comportant un carter présentant une chambre interne pour l'accumulation de liquide, ledit carter présentant un orifice d'entrée situé à une de ses extrémités pour y introduire le liquide et l'orifice d'entrée comprenent un premier alésage conduisant à un second alésage à une jonction entre les deux alésages, ledit proceédé comprenant les étapes suivantes:

amener du liquide à partir dudit premier alésage dans ledit second alésage;

prévoir les diamètres des deux alésages de telle façon que les gouttes de liquide se forment à la jonction desdits alésages, chaque goutte formant une colonne de liquide dans ledit second alèsage, et la colonne de se déplace vers le bas par gravité et s'écoule de ladite chambre;

mesurer la hauteur de chaque colonne de liquide et le nombre de colonnes par unité de temps pour fournir une mesure du débit à travers la chambre d'égouttage; et

prévoir dans ledit carter un orifice de sortie à l'extrémité opposée de celui-ci pour évacuer le liquide de ladite chambre, ledit procédé étant caractérisé en ce qu'on prévoit dans ledit carter un passage d'air menant de ladite chambre à la jonction des premier et second alésages.

10. Procédé selon la revendication 9, caractérisé en ce que la colonne de liquide se déplaçant vers le bas par gravité dans ledit second alésage (22, 122) s'écoule dans ladite chambre (32, 132; 232, 432a) avant que la colonne de liquide atteigne l'extrémité inférieure du second alésage.

0 121 578

FIG.1

FIG. 6.

**FIG. 2**

**FIG. 3**

**FIG. 5**

**FIG. 4**

FIG. 7

FIG. 8

FIG. 9